# EUROPÄISCHE PATENTSCHRIFT

(11) **EP 1 940 289 B1**
(45) Veröffentlichungstag und Bekanntmachung des Hinweises auf die Patenterteilung: **21.07.2010**
(21) Anmeldenummer: 06806285.0
(22) Anmeldetag: 14.10.2006
(51) Int. Cl.: A61B 5/151, A61B 5/00

(54) **ANALYTISCHES HILFSMITTEL MIT STERILSCHUTZ FÜR DIE LANZETTE UND TESTKAMMER**
ANALYTICAL CONTRIVANCE WITH STERILE PROTECTION FOR THE LANCET AND TEST CHAMBER
INSTRUMENT D'ANALYSE EQUIPE D'UNE PROTECTION STERILE POUR LA LANCETTE ET LA CHAMBRE D'ESSAI

(30) Priorität: 20.10.2005 EP 05022830
(43) Veröffentlichungstag der Anmeldung: 09.07.2008
(73) Patentinhaber: Roche Diagnostics GmbH, 68305 Mannheim (DE); F.HOFFMANN-LA ROCHE AG, 4070 Basel (CH)
(72) Erfinder: HINDELANG, Fritz, 67316 Carlsberg (DE); SCHWIND, Karin, 67105 Schifferstadt (DE)
(86) Internationale Anmeldenummer: PCT/EP2006/009944
(87) Internationale Veröffentlichungsnummer: WO 2007/045411

(56) Entgegenhaltungen:
- EP-A1- 1 333 756
- EP-A2- 1 466 558

## Beschreibung

Die Erfindung betrifft ein analytisches Hilfsmittel, welches eine steril geschützte Lanzette und eine analytische Testkammer enthält. Weiterhin betrifft die Erfindung ein Verfahren zur Herstellung eines solchen analytischen Hilfsmittels.

Die Untersuchung von Blutproben ermöglicht in der klinischen Diagnostik das frühzeitige und zuverlässige Erkennen von pathologischen Zuständen sowie die gezielte und fundierte Kontrolle von Körperzuständen. Die medizinische Blutdiagnostik setzt stets die Gewinnung einer Blutprobe des zu untersuchenden Individuums voraus. Während in Kliniken und bei niedergelassenen Ärzten oftmals durch eine Venenpunktion mehrere Milliliter Blut einer zu untersuchenden Person für die Analyse gewonnen werden, um damit eine Vielzahl von Labortests durchführen zu lassen, reichen für einzelne Analysen, die gezielt auf einen Parameter gerichtet sind, heutzutage oftmals wenige Mikroliter bis hin zu weniger als einem Mikroliter Blut aus. Solch geringe Blutmengen erfordern keine aufwendige und schmerzhafte Venenpunktion. Vielmehr genügt es hier, zur Blutgewinnung durch die Haut z. B. in die Fingerbeere oder das Ohrläppchen der zu untersuchenden Person mit Hilfe einer sterilen, scharfen Lanzette zu stoßen, um so einige wenige Mikroliter Blut oder weniger für die Analyse zu gewinnen. Insbesondere eignet sich diese Methode, wenn die Analyse der Blutprobe unmittelbar nach der Blutgewinnung durchgeführt werden kann.

Vor allem im Bereich des sogenannten "Home-Monitoring", also dort, wo medizinische Laien selbst einfache Analysen des Bluts durchführen, und dort insbesondere für die regelmäßige, mehrmals täglich durchzuführende Blutgewinnung durch Diabetiker für die Kontrolle der Blutglucosekonzentration, werden Lanzetten und dazu passende Geräte (sogenannte Blutentnahmegeräte, Blutlanzettenvorrichtungen oder - wie sie im Folgenden genannt werden sollen - Stechhilfen), angeboten, die eine möglichst schmerzarme und reproduzierbare Blutgewinnung ermöglichen. Zudem soll die Verwendung von Lanzetten mit Stechhilfen die psychologische Schwelle beim Stechen des eigenen Körpers senken, was vor allem für Kinder, die an Diabetes erkrankt sind und auf regelmäßige Blutglucosetests angewiesen sind, von besonderer Bedeutung ist.

Als Beispiele für Lanzetten und Stechhilfen seien die kommerziell erhältlichen Geräte (Stechhilfen) und Lanzetten Glucolet^{®} der Bayer AG und Softclix^{®} der Roche Diagnostics GmbH genannt. Solche Lanzetten und Geräte (Stechhilfen) sind z. B. Gegenstand von WO-A 98/48695, EP-A 0 565 970, US 4,442,836 oder US 5,554,166.

Die eigenhändige Blutzuckerbestimmung (das sogenannte "Home Monitoring") ist heute eine weltweit verbreitete Methode in der Diabetes-Kontrolle. Blutzuckergeräte im Stand der Technik wie z. B. AccuChek Sensor (von Roche Diagnostics) bestehen aus einem Messgerät, in das ein Testelement (Teststreifen, Sensor) eingeführt wird. Der Teststreifen wird mit einem Blutstropfen in Kontakt gebracht, der zuvor mittels einer Stechhilfe aus der Fingerbeere oder einem anderen Körperteil gewonnen wurde. Die zahlreichen Systemkomponenten (Lanzette, Stechhilfe, Teststreifen und Messgerät) benötigen viel Platz und bedingen ein relativ komplexes Handling. Inzwischen gibt es auch Systeme mit einem höheren Grad der Integration und damit einer einfacheren Handhabung. Hierzu gehören beispielsweise das AccuChek Compact (von Roche Diagnostics), das Glucometer Dex (von Bayer Diagnostics) sowie das Soft-Sense (von Medisense). Bei den beiden erstgenannten Systemen werden die Teststreifen im Messgerät magaziniert und für die Messung zur Verfügung gestellt.

Ein nächster Schritt der Miniaturisierung ist beispielsweise durch die Integration von mehreren Funktionen bzw. Funktionselementen in einem einzigen analytischen Hilfsmittel (Disposable) zu erreichen. Durch geeignete Kombination von Stechvorgang und sensorischer Analytkonzentrations-Erfassung auf einem Teststreifen lässt sich beispielsweise der Bedienablauf deutlich vereinfachen. Hierfür gibt es im Stand der Technik folgende Beispiele:
EP-B 0199 484 (Audio Bionics) beschreibt ein analytisches Hilfsmittel ("Disposables"; kurz: Dispo) mit integrierter Lanzette, die geräteseitig aktuiert wird (siehe zum Beispiel Figur 9). Mittels einer spezifischen Federlagerung (,spring-mounted lance means') wird die Lanzette nach dem Einstich wieder zurückgezogen. Das Dispo enthält ein sogenanntes Docht-Mittel ("wick means") durch das die Probenflüssigkeit von der Körperoberfläche zum Analysebereich, einem optisch auswertbaren Testfeld, geleitet wird.

In US 6,143,164 (E. Heller & Comp.) wird ein Verfahren beschrieben, bei dem eine Körperöffnung (beispielsweise ein kleiner Einstich oder Schnitt durch die Haut) erzeugt und anschließend Körperflüssigkeit in einen Sensor transportiert und dort auf die Gegenwart eines Analyten untersucht wird. US 6,143,164 offenbart hierzu ein analytisches Hilfsmittel, bei dem eine Lanzettenvorrichtung auf einem Sensor-Teststreifen angebracht ist. Der Transport der Probenflüssigkeit von der Körperöffnung zum eigentlichen Nachweiselement des Sensors erfolgt beispielsweise wider über ein "wick means" oder auch über Kapillarspalte/-kanäle.

In dem US-Patent No. 4,627,445 wird ein integriertes System beschrieben, das nur eine Öffnung sowohl für den Lanzettenaustritt wie auch für die Aufnahme des Bluts in das Gerät aufweist. Bei dieser Erfindung wird jedoch kein Sterilschutz benutzt, der sowohl die Lanzettenspitze als auch die Öffnungen steril verschließt.

Die Integration von Testelementen hat außerdem dazu geführt, dass neue Schutzelemente für die Lanzetten entwickelt wurden. Da die Benutzung der Lanzette bei einem integrierten System anders abläuft als bei manuell eingelegten Lanzetten, musste eine Möglichkeit gefunden werden, die Lanzette steril, aber trotzdem jederzeit verfügbar zu halten. Das Abziehen eines Sterilschutzes vor der Benutzung durch den Anwender, wie es bei manuell eingelegten Lanzetten üblich ist, ist bei integrierten Systemen nicht mehr nötig. Hierzu gibt es im Stand der Technik folgende Lösungsansätze:
US 2003153939 beschreibt einen Schutzmechanismus für eine Lanzette bzw. deren Lanzettenspitze, die die Spitze bis zum Stechvorgang steril hält und anschließend für eine hygienische Aufbewahrung sorgt. Der Schutzmechanismus wird beim Stechvorgang durchstochen und die Lanzette nach dem Stechvorgang wieder von dem Schutzkörper umgeben, sodass keine Verschmutzung der Umgebung durch die benutzte Lanzette gegeben ist.
US 2003050573 betrifft einen Lanzettenschutz in einem integrierten System, der die Lanzettenspitze bis zum Stechvorgang steril hält. Dabei tritt die sterile Lanzettenspitze aus einer Austrittsöffnung aus, die örtlich getrennt von der Öffnung ist, in die die Blutprobe aufgenommen wird.

Aus der EP 1 466 558 A2 ist ein Verfahren zur Herstellung eines analytischen Hilfsmittels mit Lanzetten und ein zugehöriges Testelement bekannt. Dabei wird durch eine Lanzettennadel mit einer eine Spitze aufweisenden Lanzette eine Hautöffnung erzeugt, durch die Körperflüssigkeit austritt, die dann über einen Zugangskanal in ein analytisches Testelement gesaugt werden kann. Damit zumindest die Spitze der Lanzette steril gelagert ist, wird zumindest die Lanzettenspitze von einer Schutzhülle umgeben. Dabei ist die Schutzhülle gegenüber der Lanzette ortsfest gelagert.

In der EP 1 333 756 B1 ist ein System zur Blutentnahme beschrieben. Dieses System weist eine Antriebseinheit und eine Stecheinheit, in der sich eine Lanzette mit einer Nadel befindet, auf. Um zumindest die Nadelspitze steril zu lagern, ist sie in einem Material, vorzugsweise in einem Elastomer, angeordnet, das die Nadelspitze so dicht umgibt, dass keine Kontamination oder eine Verformung der Nadelspitze auftreten kann. Das System ist als reine Stechhilfe ohne analytische Komponenten ausgebildet.

Ein Manko bekannter Dispos ist die mangelnde Integration verschiedener Funktionen wie Lanzettensterilschutz, Sterilschutz des Testraumes und möglichst kurzen Wegen für den Bluttransport ohne, dass der Patient einwirken muss. So gibt es zwar Lösungen für die Einzelaspekte, aber keine Lösung für die Kombination all dieser Anforderungen. So wird beispielsweise im Stand der Technik bei Versiegelung von Gehäuseöffnungen das Versiegelungsmaterial durchstochen und da das Versiegelungsmaterial nicht verschiebbar ist, muss eine zusätzliche Öffnung generiert werden, um die Probe aufzunehmen. Dadurch ist für integrierte Systeme, die mit Sterilschutz arbeiten, ein komplizierter Aufbau nötig, der eine räumliche Trennung von Stechvorgang und Blutaufnahme vorsieht. Dadurch ist der Patient gezwungen aktiv in den Blutentnahmeprozess einzugreifen. Dies bedeutet einen großen Komfortverlust und ist insbesondere für Patienten mit geringem Sehvermögen sehr kompliziert.

Die Aufgabe der vorliegenden Erfindung war es daher, die Nachteile des Standes der Technik, zu beseitigen. Insbesondere war es die Aufgabe der vorliegenden Erfindung, analytische Hilfsmittel (oder synonym dazu: "Disposables", kurz: "Dispos") zur Verfügung zu stellen, die die Nachteile des Standes der Technik nicht zeigen. Ganz besonders sollte die Sicherstellung der Lanzettensterilität für den Zeitraum der Aufbrauchfrist durch das erfindungsgemäße Dispo bei gleichzeitiger Integration von Lanzette und Testelement gewährt werden. Dabei sollte ein möglichst kurzer Weg für die zu analysierende Flüssigkeit von der Dispo-Öffnung zum Testelement gewährleistet sein. Für den Patienten sollte dieses System einen hohen Bedienkomfort aufweisen, in dem der Patient nach dem Stechen sich nicht weiter um die Blutgewinnung kümmern muss.

Weiterhin ist es die Aufgabe der vorliegenden Erfindung, analytische Hilfsmittel mit Lanzetten bereitzustellen, bei denen zumindest die Lanzettennadelspitze im unbenutzten Zustand bis unmittelbar vor der Benutzung steril, das heißt keimfrei, gehalten wird und im benutzten Zustand hygienisch aufbewahrt werden kann. Idealerweise sollte diese Aufgabe gelöst werden, ohne dass der Benutzer für die hygienische Aufbewahrung separate Maßnahmen zu ergreifen hat. Zudem sollte der Benutzer vor unbeabsichtigter Verletzung mit der Lanzette, insbesondere der benutzten Lanzette geschützt sein. Schließlich sollte vorzugsweise ein einfacher Probentransfer von der Stelle der Blutgewinnung zur Stelle der Blutuntersuchung möglich sein.

Diese Aufgaben werden durch den Gegenstand der Erfindung, wie er in den unabhängigen Patentansprüchen charakterisiert wird, gelöst. Bevorzugte Ausführungsformen sind Gegenstand der abhängigen Ansprüche.

Ein erster Gegenstand der Erfindung ist ein analytisches Hilfsmittel, das eine Lanzette enthält. Die Lanzette weist als bevorzugte Bestandteile eine Lanzettennadel mit einer Spitze und einer Schutzkappe auf, die die Lanzettennadel zumindest im Bereich der Spitze vollständig umgibt. Die Lanzettennadel ist dabei relativ zur Schutzkappe verschiebbar. Die Schutzkappe kann dabei aus verschiedenen Materialien bestehen die durchstechbar sind und in die die Spitze der Lanzettennadel eingebettet ist. Das analytische Hilfsmittel enthält weiterhin ein analytisches Testelement, bestehend aus einer Kammer, die ein Nachweiselement mit einem Reagenzsystem enthält. Diese Kammer weist eine Öffnung auf, durch die die Lanzette beim Stechvorgang bewegt wird. Diese Öffnung wird durch die Schutzkappe der Lanzettennadel verschlossen.

Schließlich ist ein Verfahren zur Herstellung solcher analytischer Hilfsmittel Gegenstand der Erfindung.

Die erfindungsgemäße Lösung besteht vorzugsweise aus einem miniaturisierten Dispo, bei dem die drei Funktionen Stechen, Bluttransfer von der durch das Stechen erzeugten Wunde zum Testelement, und Nachweis des Analyten, in einem Körper zusammengefasst sind.

Der Grundkörper des erfindungsgemäßen analytischen Hilfsmittels besteht aus einem starren Kunststoffkörper vorzugsweise mit Hohlräumen, dessen äußere Form vorzugsweise zum Zwecke des Verschlusses einer Gehäuseöffnung entsprechend angepasst ist. In diesem Kunststoff wird eine Lanzettennadel derart eingebettet, dass ihre Spitze vorzugsweise nicht über die Vorderkante des Grundkörpers herausragt. Der Grundkörper kann daher auch als Schutzkappe bezeichnet werden. Der Grundköper besitzt in einer bevorzugten Ausführungsform Stege, die zur Fixierung der Nadel im Grundkörper und zur Führung während der Stechbewegung dienen. Vorzugsweise ist der überwiegende Teil der Nadel jedoch nicht mit dem Grundkörper verbunden, um die Reibungskräfte bei der Stechbewegung zu minimieren. Bevorzugt werden die Kontaktflächen zwischen Nadel und Schutzkappe minimiert und geeignet vorbehandelt, beispielsweise silikonisiert.

Das analytische Hilfsmittel besteht aus einem Gehäuse in dem sich die Lanzettennadel mit Schutzkappe sowie das Testelement befinden. Optional ist bei elektrochemischer Messung mindestens eine Elektrode in der Testkammer vorhanden Das Gehäuse besteht in einer bevorzugten Ausführungsform aus 2 Gehäuseteilen. Diese Gehäuseteile können durch Spritzgussverfahren aus verschiedenen Kunststoffen hergestellt werden. Eine nicht abschließende Auswahl an Polymeren sind Polyester, Polycarbonat, Polyvinylchlorid, Polymethylmethacrylat, Co-Polyester sowie Gemische daraus. Bei optischer Auswertung des Testelementes ist ein Teil des Gehäuses aus durchsichtigem Material.

Die erfindungsgemäßen Lanzetten sind vorzugsweise für den einmaligen Gebrauch konzipiert und können daher auch als Einweg-Blutlanzetten oder Wegwerf-Blutlanzetten bezeichnet werden. Die Lanzette der Erfindung beinhaltet eine Nadel (Lanzettennadel) mit einer Spitze. Die Nadel ist in der Regel mehrere Millimeter (mm) bis wenige Zentimeter (cm) lang und weist eine längliche Gestalt auf. Typischerweise besitzen Nadeln eine zylindrische Gestalt, da diese Nadelform besonders gut herstellbar ist; es sind jedoch auch Nadelformen mit abweichender Formgebung möglich. Der Spitzenbereich der Nadel beinhaltet die Nadelspitze, die beim bestimmungsgemäßen Gebrauch der Lanzette in Gewebe eingestochen wird. Die Spitze der Lanzettennadel ist folglich der Teil der Lanzette, der mit der Haut des zu stechenden Individuums in Berührung kommt, diese ggf. verletzt und so den Ausfluss einer Körperflüssigkeit, insbesondere Blut oder interstitieller Flüssigkeit, verursacht.

Die Spitze der Lanzettennadel kann beispielsweise rotationssymmetrisch sein, wie dies im Allgemeinen bei Stecknadeln der Fall ist. Es hat sich jedoch als vorteilhaft herausgestellt, wenn man an der Nadelspitze einen oder mehrere Schliffe anbringt. Die hierbei entstehenden, zur Längachse der Nadel geneigten, in einer Spitze zulaufenden Kanten dienen beim Einstich als scharfe Schneide und gestalten den Einstichvorgang schmerzärmer, als dies mit ungeschliffenen Nadeln der Fall ist.

Die Lanzettennadel der erfindungsgemäßen Lanzette ist aus einem Material gefertigt, das ausreichend hart ist, um eine mechanische Beanspruchung während des Einstichvorgangs, der Bearbeitungsschritte oder eventuell sonstige auftretende Beanspruchungen ohne Deformation zu überstehen. Weiterhin muss das Material so beschaffen sein, dass während des Einstichvorgangs keine Partikel abbrechen oder sich ablösen. Schließlich muss das Nadelmaterial auch so bearbeitbar sein, dass die Nadelspitze ausreichend spitz und die Kanten der Nadelspitze gegebenenfalls ausreichend scharf geschliffen werden können. Gut geeignete Materialien für die Lanzettennadel sind vor allem Metalle und von diesen insbesondere Edelstähle. Wenn auf die Eigenschaft der Lanzette als Füllstandsmesser und Gegenelektrode verzichtet wird, sind jedoch auch Nadeln aus Silizium, Keramik oder Kunststoffen denkbar. Edelstahlnadeln sind besonders bevorzugt.

Erfindungsgemäß ist in einer Ausführungsform zumindest die Spitze der Lanzettennadel der erfindungsgemäßen Lanzette von der Schutzkappe umgeben. Wesentlich ist dabei, dass die Schutzkappe im Bereich der Spitze der Lanzettennadel aus einem Material besteht das für die Lanzettenspitze durchstoßbar ist. Ist die Schutzkappe aus einem elastischen Material gefertigt, so umgibt sie die Lanzettenspitze bevorzugterweise vollständig. Somit ist die Lanzettenspitze von der Umgebung abgeschlossen. Das elastische Material der Schutzkappes, welches in unterschiedlichen Ausführungsformen die Schutzkappe vollständig oder nur teilweise bilden kann, zeichnet sich dadurch aus, dass es weich, verformbar und von der Lanzettennadel mit ihrer Spitze durchstoßbar ist, ohne die Spitze zu verletzen. Im Falle einer nicht elastischen Schutzkappe wird die Lanzettenspitze vorzugsweise so von der Schutzkappe umgeben, dass ein Hohlraum zwischen Lanzettenspitze und der Schutzkappenwandung vorliegt. Die Wandungsstärke des Schutzkappenmaterials ist dabei so ausgelegt, dass auch hier keine Verformung und Abnutzung der Schliffkanten der Lanzettenspitze beim Durchstoßen auftritt.

Beim Stechvorgang wird die Lanzettennadel entlang ihrer Längsachse relativ zur Schutzkappe bewegt und tritt mit ihrer Spitze durch die Schutzkappe aus dem Gehäuse aus, um so zur Blutgewinnung in die Haut des zu untersuchenden Individuums einstechen zu können.

Das elastische Material der Schutzkappe, welches die Spitze der Lanzettennadel vollständig umschließt, gewährleistet die Sterilität der Lanzettennadelspitze vor deren Benutzung, vorzugsweise bis unmittelbar vor deren Benutzung. Das elastische Material ist folglich keimdicht für das Eindringen oder Entweichen von Keimen im unbenutzten Zustand der Lanzettennadel. Zudem stellt das elastische Material einen mechanischen Schutz für die Lanzettennadelspitze dar und verhindert so auch ein unbeabsichtigtes Verletzen an der Lanzettennadelspitze.

Als elastisches Material für die Schutzkappe der vorliegenden Erfindung haben sich Gummi, Kautschuk, Silikon, Elastomere und insbesondere thermoplastische Elastomere als geeignet herausgestellt. Diese weisen die für die vorliegende Erfindung wesentlichen Eigenschaften auf: sie sind weich, verformbar, von der Lanzettennadel zu durchstoßen, ohne die Spitze zu verletzen, und schließen sich dicht um die unbenutzte Lanzettennadelspitze. Des Weiteren können Sie für Spritzgussprozesse verwendet werden, die eine Massenfertigung von Lanzetten in großen Stückzahlen ermöglichen.

Thermoplastische Elastomere, die auch Elastoplaste oder Thermoplaste oder thermoplastische Kautschuke genannt werden, besitzen im Idealfall eine Kombination der Gebrauchseigenschaften von Elastomeren und den Verarbeitungseigenschaften von Thermoplasten. Thermoplastische Elastomere sind beispielsweise Styrol-Oligoblock-Copolymere (sogenannte TPE-S), thermoplastische Polyolefine (TPE-O), thermoplastische Polyurethane (TPE-U), thermoplastische Copolyester (TPE-E) und thermoplastische Copolyamide (TPE-A). Insbesondere haben sich beispielsweise thermoplastische Elastomere auf der Basis von Styrol-Ethylen-Butylen-StyrolPolymeren (SEBS-Polymere, z. B. Evoprene® von Evode Plastics oder Thermolast K von Gummiwerk Kraiburg GmbH) als geeignet erwiesen.

Während des Stechvorgangs wird die Lanzettennadel relativ zur Schutzkappe bewegt. Die Schutzkappe wird dabei vorzugsweise von der Stechhilfe oder dem Stechgerät in seiner Position fixiert. Die Lanzettennadel kann zum Zwecke ihres Antriebs besonders ausgeformt sein, beispielsweise kann sie einen Nadelkopf an dem der Spitze entgegengesetzten Ende besitzen, oder zusätzlich zur Schutzkappe, der die Spitze umschließt, einen weiteren Lanzettenkörper bzw. Lanzettenhaltung aufweisen, der von einem Antriebselement der Stechhilfe ergriffen werden kann. Die Ausformung der Nadel oder der zusätzliche Lanzettenhaltung können in geeigneter Weise mit einer entsprechenden Antriebsvorrichtung im Stechgerät (Stechhilfe) wechselwirken. Allgemein können solche Mittel als Antriebsvorrichtung der Nadel bezeichnet werden. Solche Antriebsvorrichtungen sind dem Fachmann hinreichend bekannt, wie zum Beispiel aus den Patentanmeldungen US 6,783,537B1 oder EP 1 336 375.

Zur Erhöhung der Stabilität des elastischen Materials ist es möglich, dieses mit einem steifen Material, beispielsweise einem steifen Kunststoffmaterial, zu verbinden. Das elastische Material kann dabei beispielsweise auf seiner Außenseite, die nicht mit der Spitze der Lanzettennadel in Berührung kommt, mit einer Schicht eines steifen Materials, beispielsweise eines steifen Kunststoffs, stabilisiert sein. Es ist auch möglich, den Lanzettenschutz nur im Bereich der Lanzettennadelspitze aus einem elastischen Material zu fertigen, im Übrigen die Umhüllung der Lanzette jedoch aus herkömmlichen, steifen Kunststoffen zu fertigen. Dabei können das elastische Material und das steife Material miteinander verklebt sein oder durch einen Spritzgussprozess, beispielsweise einen Zweikomponenten-Spritzgussprozess, miteinander verbunden sein. Das steife Material der Lanzettenumhüllung sorgt dabei für eine mechanische Stabilisierung des elastischen Materials während des Stechvorgangs und erleichtert die Fixierung des elastischen Teils der Schutzkappe während des Stechvorgangs durch die Stechhilfe. Das steife Material kann auch Teil des Testelements sein, beispielsweise eines Kapillarspalttestelements wie es in WO 99/29429 beschrieben ist. In einer weiteren Ausführungsform kann die ganze Schutzkappe aus einem steifen Kunststoffmaterial bestehen.

Der Patient kommt beim Stechvorgang mit der Schutzkappe nicht oder nur partiell in Berührung. Vielmehr legt der Patient seinen Finger auf die Öffnung des Gehäuses, die sich bevorzugterweise nicht auf der Seite des Antriebs und der Ankopplung des Dispos an das Messgerät befindet. Nach Anlegen des Fingers kann der Patient einen Mechanismus auslösen, der die Bewegung der Lanzette aus dem Ruhezustand in einen Aktuationszustand und wieder zurück in den Ruhezustand bewirkt. Dabei bewegt sich die Lanzette mit ihrem distalen Ende, also der Spitze, für eine kurze Zeitspanne durch die Öffnung aus dem Gehäuse heraus. Auf ihrem Weg zurück in das Gehäuse, zieht sie mittels einer Fangeinrichtung, die an den Lanzettenkörper gekoppelt oder mit ihm verbunden ist, die Schutzkappe, die auch während des Stichs im Gehäuse verbleibt, mit zurück.

Der Bluttransfer von der Wunde/Einstichstelle der Lanzette zum Messort wird erfindungsgemäß auf folgende Art realisiert: Vorzugsweise wird der Bluttransfer ohne Zutun des Benutzers des erfindungsgemäßen Dispos, quasi "automatisch" bewerkstelligt. Zu diesem Zweck kann das Dispo Mittel zum Probenflüssigkeitstransport aufweisen. Vorzugsweise sind diese Mittel kapillaraktiv, beispielsweise als Spalte oder Kanäle in einem starren Grundkörper oder als saugfähige Matrixmaterialien ausgebildet. Möglich ist auch eine Kombination dieser beiden prinzipiellen Möglichkeiten, beispielsweise dass das Blut zunächst über einen Kapillarkanal geführt, von einem saugfähigen Matrixmaterial übernommen und in eine Testkammer abgegeben wird.

Als saugfähige Matrixmaterialien im Sinne dieser Erfindung haben sich insbesondere saugfähige Vliese, Papiere, Dochte, oder Gewebe als geeignet erwiesen.

In einer bevorzugten Variante enthält der Grundköper, bevorzugt die Testkammer, des analytischen Hilfsmittels das Mittel zum Probenflüssigkeitstransport. Dies kann ein saugfähiger, in die Kammer eingelassener Docht oder - was bevorzugt ist - ein ausgeformter Kapillarspalt sein, der gleichzeitig als Austrittsöffnung für die Lanzette dient. Dadurch wird erreicht, dass das Dispo zur Blutaufnahme nicht verschoben werden muss. Die Geometrie der Einlaßöffnung ist derart gestaltet, dass sich der gebildete Blutstropfen möglichst leicht auffangen und eingeben lässt, beispielsweise als Trichter oder Kerbe. Die Kapillarwirkung sorgt dann für das Ansaugen der benötigten Blutmenge, die deutlich unterhalb von 1 Mikroliter liegen kann. Das Blut gelangt auf diesem Wege in die Kammer zum Testfeld und reagiert dort mit der Testchemie, wobei ein auswertbares elektrisches Signal oder eine Farbänderung erzeugt wird. Der Kapillarspalt kann beim Spritzgießen in den Kunststoff eingeformt werden oder nachträglich in den Kunststoffkörper eingebracht werden, beispielsweise geprägt oder gefräst. Besonders bevorzugt wird das Einsaugen des Probentropfens in den kapillaren Kanal dadurch erreicht, dass die durch die Aussparung frei liegende Fläche hydrophiliert ist und zumindest in Richtung des kapillaren Transportkanals direkt an eine kapillaraktive Zone grenzt.

Hydrophile Oberflächen sind in diesem Zusammenhang Wasser anziehende Flächen. Wässrige Proben, darunter auch Blut, spreiten auf solchen Oberflächen gut. Solche Flächen sind unter anderem dadurch charakterisiert, dass an der Grenzfläche ein Wassertropfen auf ihnen einen spitzen Rand- oder Kontaktwinkel ausbildet. Im Gegensatz dazu wird auf hydrophoben, das heißt Wasser abweisenden Oberflächen, an der Grenzfläche zwischen Wassertropfen und Oberfläche ein stumpfer Randwinkel ausgebildet. Die Bereitschaft einer Kapillare, eine Flüssigkeit aufzusaugen, geht mit der Benetzbarkeit der Kanaloberfläche mit der Flüssigkeit einher. Für wässrige Proben bedeutet dies, dass eine Kapillare aus einem Material gefertigt werden sollte, dessen Oberflächenspannung nahe an die von Wasser heranreicht (72 mN/m) oder diesen Wert übertrifft.

Ausreichend hydrophile Materialien zum Aufbau einer Kapillare, die schnell wässrige Proben aufsaugt, sind beispielsweise Glas, Metall oder Keramik. Für den Einsatz in Testträgern sind diese Materialien jedoch weniger geeignet, da sie einige gravierende Nachteile aufweisen. Dies ist beispielsweise die Bruchgefahr bei Glas oder Keramik, oder Veränderung der Oberflächeneigenschaften mit der Zeit bei zahlreichen Metallen. Üblicherweise werden deshalb zur Fertigung von Testelementen Kunststofffolien oder -formteile eingesetzt. Die verwendeten Kunststoffe übertreffen dabei in der Regel kaum eine Oberflächenspannung von 45 mN/m. Selbst mit den, relativ betrachtet, hydrophilsten Kunststoffen wie beispielsweise Polymethylmethacrylat (PMMA) oder Polyamid (PA) lassen sich - wenn überhaupt - nur sehr langsam saugende Kapillaren aufbauen. Kapillaren aus hydrophoben Kunststoffen wie beispielsweise Polystyrol (PS), Polypropylen (PP) oder Polyethylen (PE) saugen im Wesentlichen keine wässrigen Proben. Hieraus ergibt sich die Notwendigkeit, Kunststoffe für die Verwendung als Konstruktionsmaterial für Testelemente mit kapillaraktiven Kanälen hydrophil auszustatten, das heißt zu hydrophilieren.

Idealerweise wird die Hydrophilierung der Oberfläche des kapillaren Kanals dadurch erreicht, dass zu seiner Fertigung ein hydrophiles Material eingesetzt wird, das jedoch die Probenflüssigkeit selbst nicht oder nicht wesentlich aufzusaugen vermag. Wo dies nicht möglich ist, kann die Hydrophilierung einer hydrophoben oder nur sehr wenig hydrophilen Oberfläche durch geeignete Beschichtung mit einer stabilen, gegenüber dem Probenmaterial inerten, hydrophilen Schicht erreicht werden, beispielsweise durch kovalente Bindung von photoreaktiv ausgerüsteten, hydrophilen Polymeren auf eine Kunststoffoberfläche, durch Aufbringen netzmittelhaltiger Schichten oder durch Beschichtung von Oberflächen mit Nanokompositen mittels Sol-Gel-Technologie. Darüber hinaus ist es möglich, durch thermische, physikalische oder chemische Behandlung der Oberfläche eine gesteigerte Hydrophilie zu erzielen. Hierzu zählt z.B. auch die Plasmabehandlung der Oberfläche. Dies geschieht beispielsweise mit hochenergetischem Sauerstoff oder anderen polarisierenden Medien.

Ganz besonders bevorzugt wird die Hydrophilierung durch die Verwendung von dünnen Schichten oxidierten Aluminiums erreicht. Diese Schichten werden entweder direkt auf die gewünschten Bauteile des Testelements aufgebracht, beispielsweise durch Vakuumbedampfen der Werkstücke mit metallischem Aluminium und anschließende Oxidation des Metalls, oder in Form von Metallfolien oder metallbeschichteten Kunststoff-Folien für den Testträgeraufbau verwendet, die ebenfalls zur Erzielung der erwünschten Hydrophilie oxidiert werden müssen. Metallschichtdicken von 1 bis 500 nm sind dabei ausreichend. Die Metallschicht wird anschließend zu Bildung der oxidierten Form oxidiert, wobei sich neben der elektrochemischen, anodischen Oxidation vor allem die Oxidation in Gegenwart von Wasserdampf oder durch Kochen in Wasser als besonders geeignete Methoden herausgestellt haben. Die so erzielten Oxidschichten sind je nach Methode zwischen 0,1 und 500 nm, bevorzugt zwischen 10 und 100 nm dick. Größere Schichtdicken sowohl der Metallschicht als auch der Oxidschicht sind zwar prinzipiell praktisch realisierbar, zeigen aber keine weiteren vorteilhaften Wirkungen.

In einer bevorzugten Ausführungsform enthält das Nachweiselement des erfindungsgemäßen analytischen Testelements alle für die Nachweisreaktion des Zielananlyten in der Probe notwendigen Reagenzien und gegebenenfalls Hilfsstoffe. Das Nachweiselement kann auch nur Teile der Reagenzien oder Hilfsstoffe enthalten. Dem mit der Technik von analytischen Testelementen oder diagnostischen Testträgern vertrauten Fachmann sind solche Reagenzien und Hilfsmittel bestens bekannt. Für Analyten, die enzymatisch nachzuweisen sind, können beispielsweise Enzyme, Enzymsubstrate, Indikatoren, Puffersalze, inerte Füllstoffe und dergleichen mehr im Nachweiselement enthalten sein. Das Nachweiselement kann aus einer oder mehreren Schichten aufgebaut sein und gegebenenfalls einen inerten Träger, bevorzugt auf der Seite des Nachweiselements, die nicht mit der Probe in Kontakt gebracht wird, enthalten. Für den besonders bevorzugten Fall, dass die Nachweisreaktion zu einer beobachtbaren Farbveränderung führt, worunter in diesem Zusammenhang entweder die Änderung einer Farbe, das Entstehen einer Farbe oder das Verschwinden von Farbe verstanden werden soll, ist sicherzustellen, dass der Träger durch geeignete Maßnahmen eine visuelle oder optische Beobachtung der Nachweisreaktion zulässt. Dazu kann das Trägermaterial des Nachweiselements selbst durchsichtig sein, beispielsweise eine durchsichtige Kunststoff-Folie, wie beispielsweise Polycarbonatfolie, oder auf der Detektionsseite eine durchsichtige Aussparung besitzen. Neben Nachweisreaktionen, die zu Farbveränderungen führen, sind dem Fachmann auch andere Nachweisprinzipien bekannt, die mit dem beschriebenen Testelement realisiert werden können, beispielsweise elektrochemische Sensoren.

Beim elektrochemischen Nachweis des Analyten befindet sich mindestens eine Elektrode in der Testkammer. Die Elektrode wird über einen Kontakt am Dispo mit dem Messgerät verbunden. Hierbei kann die Lanzette als zweite Elektrode verwendet werden. Die Lanzette wird vorzugsweise über einen Federkontakt mit dem Messgerät verbunden, aber auch starre Kontakte der Lanzette mit dem Messgerät sind möglich. Die Messung kann sowohl mit Gleichstrom wie auch mit Wechselstrom durchgeführt werden: Es besteht darüber hinaus die Möglichkeit, die Lanzette als Füllstandskontrolle zu benutzen. Dies kann ebenfalls durch einen Federkontakt ermöglicht werden, der nach Benutzung der Lanzette, die Lanzette mit dem Messgerät verbindet und bei Kontakt der Lanzette mit der eintretenden Flüssigkeit eine Stromstärken - oder Spannungsänderung auftritt.

Die Testkammer beinhaltet sowohl die Lanzettenspitze mit Schutzkappe sowie die nötigen Nachweischemikalien und bei elektrochemischem Nachweis die Elektroden. Die Testkammer ist in ihrer Größe so ausgelegt, dass ein minimales Volumen der Testflüssigkeit benötigt wird. Dies liegt idealer Weise unterhalb einem µl. Zum optimalen Transport der Flüssigkeit in die Testkammer über die Austrittsöffnung, werden vorzugsweise hydrophile Materialien zur Herstellung der Testkammer genutzt.

Die Testkammer kann verschiedene Geometrien besitzen. So kann die Kammer Würfel- oder Quaderförmig sein. Die Kammer kann aber auch die Form einer runden oder ovalen Habkugel einnehmen. Die Kammer besitzt mindestens eine Öffnung, durch die die Lanzette bei Aktuation austritt. Im Ruhezustand, vor der Benutzung, ist diese Öffnung durch die Schutzkappe, die die Lanzettenspitze umgibt, verschlossen. Nach Aktuation der Lanzette wird eine weitere Belüftung der Kammer benötigt, um Kapillarwirkung in die Kammer zu erzielen. Dies kann durch eine zweite Öffnung der Kammer gewährleistet werden oder durch ein Belüftungsloch in der Lanzettenhalterung, die vor Benutzung der Lanzette durch eine Folie oder sonstige Versieglung verschlossen ist. Die Geometrie und das Volumen der Kammer sind abhängig von der Anzahl der Elektroden in der Kammer. Die Anzahl der Elektroden richtet sich nach der Art der Verwendung des Hilfsmittels. Für die elektrochemische Analyse ist mindestens eine Elektrode in der Testkammer, wobei die Lanzette als Gegen- oder Bezugselektrode benutzt werden kann. Dies kann durch weitere Elektroden ergänzt werden. Durch das Hinzufügen weiterer Elektroden besteht die Möglichkeit, mehr als einen Analyten unabhängig voneinander zu bestimmen. Bei diesen Analyten kann es sich z.B. um Blutbestandteile wie Cholesterin, Triglyceride, Gerinnungsfaktoren und andere Blutparameter handeln. Das Volumen der Kammer liegt zwischen 100 nl und 1000 nl, kann aber bei Verwendung einer Vielzahl von Elektroden auch größer sein. In einer bevorzugten Ausführungsform liegt das Volumen zwischen 300 nl und 600 nl, besonders bevorzugt bei 500 nl. Die Elektroden sowie Ihre Kontakte sind aus einem leitfähigen Material, wie leitende Kunststoffe oder Metall. Bei der Verwendung der Lanzettenspitze als Elektrode bestehen sowohl die Elektroden und deren Kontakte als auch die Lanzette aus leitfähigem Material, wie z. B. Aluminium, Blei, Eisen, Gallium, Gold, Indium, Iridium, Kohlenstoff (wie Graphite), Kobalt, Kupfer, Magnesium, Nickel, Niob, Osmium, Palladium, Platin, Quecksilber (als Amalgam), Rhenium, Rhodium, Selen, Silizium (wie hoch dotiertes polykristallines Silizium), Silber, Tantal, Titan, Uran, Vanadium, Wolfram, Zinn, Zink, Zirkonium, Mischungen sowie Legierungen daraus, Oxide oder Metallgemische der aufgeführten Elemente. Bevorzugt beinhalten die Kontakte und Elektroden Gold, Platin, Palladium, Iridium oder Gemische bzw. Legierungen aus diesen Metallen. Dabei können die Kontakte aus einem anderen Material sein als die Elektroden. Genauso kann die Lanzettenspitze eine andere Zusammensetzung aufweisen als der Rest des Lanzettenkörpers. Eine bevorzugte Ausführungsform ist eine Silber/Silberhalogenid Elektrode als Referenzelektrode sowie eine (z.B., siebgedruckte) Graphitelektrode als Arbeitselektrode.

Bei optischer Detektion der chemischen Umsetzung in der Testkammer, besitzt das Gehäuse des Gerätes mindestens eine optisch transparente Gehäusewand, die entweder unterhalb oder oberhalb der Testchemie angebracht ist. Für ausschließlich optische Messung braucht in der Testkammer hierbei keine Elektrode vorhanden sein, eine Kombination aus elektrochemischer und optischer Messung ist ebenfalls möglich. Zur optischen Detektion wird ein Lichtstrahl auf das Testfeld gerichtet, dessen Wechselwirkung mit der Flüssigkeit entweder in Reflektion oder Transmission gemessen werden kann. Im Falle der Transmissionsmessung wird sowohl auf der Seite der Einstrahlung als auch auf der Detektionsseite eine optisch transparente Gehäusewand verwendet. Bei der optischen Detektion wird ein Nachweiselement, das Bestandteil des Testelements ist und die Nachweisreagenzien beinhaltet, auf der transparenten Oberfläche der Testkammer befestigt. Bei der Reaktion der Testflüssigkeit mit dem Testreagenz im Nachweiselement, ändert sich ein optisches Merkmal, was mit Hilfe eines optischen Moduls detektiert wird. Dieses optische Modul kann ein Photosensor oder ein Photomultiplier sein, oder sonst eine optische Sensoreinheit bekannt aus dem Stand der Technik. Die Strahlungsquelle kann ebenfalls eine im Stand der Technik bekannte sein. Bei der optischen Messung kann ebenfalls die zurückgezogene Lanzette als Füllstandsmesser benutzt werden.

Die Fangeinrichtung, die zum Zurückziehen der Schutzkappe und/oder einer weiteren Dichtung dient, kann verschiedene Ausführungsformen besitzen. Eine erfindungsgemäße Ausführungsform ist das Aufrauen der Lanzettenoberfläche an geeigneter Stelle des Lanzettenkörpers. Hierdurch entsteht beim Kontakt dieser aufgerauten Fläche mit dem bzw. den Dichtungskörpern eine ausreichende Reibungswirkung, sodass beim Zurückziehen der Lanzette der bzw. die Dichtungskörper daran haften bleiben.

Eine zweite erfindungsgemäße Ausführungsform ist ein Fanghaken, der wiederum verschiedene Ausführungsformen besitzen kann. Das Prinzip ist das Verhaken der Dichtungskörper an den Widerhaken des Lanzettenkörpers beim maximalen Austritt der Lanzette. Somit wird der bzw. die Dichtungskörper mit der Lanzette zurückgezogen. Der Fanghaken kann dabei aus borstenförmigen Ausformungen oder Haken aus der Lanzette bestehen, die Ihre Ausrichtung zum proximalen Ende der Lanzette haben. Diese Haken können aus verschiedenen vorzugsweise unverformbaren Materialien wie Metall, Keramik oder Polymeren sein, bevorzugt aus Metall.

Eine weitere Ausführungsform für die Fangeinrichtung ist ein Fanghaken, der sich über die Dichtungskörper schiebt und diese umschließt. Durch das Umschließen der Dichtungskörper wird das Zurückziehen der Dichtungen bewirkt, wenn die Lanzette nach Aktuation wieder zurückgezogen wird. Dieser Fanghaken ist mit dem Lanzettenkörper über eine Halterung verbunden. Der Fanghaken besitzt mindestens einen Arm der sich in Richtung Lanzettenspitze erstreckt und mit einem Haken an dem distalen Ende ausgerüstet ist. Der Haken stülpt sich bei maximaler Auslenkung der Lanzette über den oder die Dichtungskörper. Zum sicheren Einfangen der Dichtungen wird eine bevorzugte Ausführungsform mit mindestens zwei Armen gewählt.

Dieser Fanghaken oder auch die aufgeraute Oberfläche an der Lanzette, zieht sowohl die Schutzkappe, wie auch weitere vorhandene Dichtungen in das Gehäuse zurück Durch die zurückgezogenen Dichtungen wird sowohl die Austrittsöffnung als auch eine weitere Öffnung zur Belüftung der Testkammer geöffnet. Diese Abdichtung der Testkammer verhindert zu dem, dass die Elektroden und die Testreagenzien bei der Lagerung verunreinigt werden. Die Schutzkappe verhindert zudem einen ungewollten, vorzeitigen Kontakt des Patienten mit der Lanzette.

Allgemein lässt sich die Funktion des erfindungsgemäßen Disposables wie folgt schildern:
1. Das Dispo wird in die Aufnahmevorrichtung eines (Blutzucker-) Messgerätes gesteckt und damit fixiert. Für magazinierte Dispos wird ein Magazin von Dispos in das Messgerät eingelegt.
2. Der Antriebsmechanismus der Stecheinheit wird gespannt und koppelt an die Antriebsvorrichtung des Dispos an.
3. Die Kontakte des Dispos treten bei der Fixierung des Dispos an das Messgerät mit der elektrischen Versorgung in dem Messgerät in Kontakt.
4. Der Benutzer kontaktiert die Öffnung des analytischen Hilfsmittels mit dem Finger oder der Körperstelle, an der die Messung durchgeführt werden soll.
5. Beim Auslösen des Stechvorgangs wird die Nadel nach vorne bewegt und tritt dabei durch die Schutzkappe mit hoher Geschwindigkeit aus der Öffnung. Der gesamte Stechvorgang spielt sich im Bereich weniger Millisekunden ab.
6. Nach erfolgtem Einstich in die Haut wird die Nadel wieder zurückgezogen. Dabei zieht eine Fangeinrichtung, die sich an der Lanzette befindet, die Schutzkappe und ggf. weitere Dichtungen mit sich. Der Antrieb wird gegebenenfalls wieder abgekoppelt.
   a) bei Verwendung der Lanzette als Gegenelektrode wird die Lanzette mit einem zusätzlichen Federkontakt verbunden, der in die Antriebseinheit integriert sein kann.
7. Der Benutzer kontaktiert (weiterhin) die Öffnung des Hilfsmittels an seiner Aufnahmevorrichtung, so dass die Ansaugöffnung (z. B. Kapillare) einen Bluttropfen aufnehmen kann.
8. Durch die Saugwirkung der Mittel zum Probenflüssigkeitstransport, wird das Blut im Dispo an eine Stelle in der Testkammer transportiert, in der sich ein Testelement mit Nachweiselement befindet.
9. Im Nachweiselement findet eine Reaktion der nachzuweisenden Blutbestandteile mit den Nachweisreagenzien statt, die beispielsweise mittels photometrischer oder elektrochemischer Detektion detektiert wird.
10. Aus den elektronischen Daten wird ein Messergebnis berechnet und dem Benutzer optisch oder akustisch angezeigt.
11. Bei magazinierten Dispos wird das Magazin weitergetaktet. Bei Einmal-Dispos wird das benutzte Dispo ausgeworfen oder per Hand entfernt.

Messgeräte die dem Fachmann bekannt sind, die verschiedene Merkmale wie Algorithmen zur Auswertung sowie verschiedene Energiequellen zur Versorgung des Dispos besitzen sind in den folgenden Anmeldungen beschrieben U.S 4,963,814; U.S. 4,999,632; U.S. 4,999,582; U.S. 5,243,516; U.S. 5,352,351; U.S. 5,366,609; U.S. 5,405,511; U.S. 5,438,271.

Die Herstellung eines erfindungsgemäßen Disposables ist prinzipiell in den folgenden einfachen Schritten realisierbar:
(1) Spritzgießen der Gehäuseteile.
(2) Spritzgießen des Grundköpers (Schutzkappe) inklusive Einbettung der Lanzettennadel (ggf. mit Erzeugung des "Nadelkopfes", d. h. der von einem Stechgerät greifbaren Verdickung).
(3) Umspritzung der Nadelspitze mit Weichkunststoff.
(4) Sterilisation der "Rohdispos", beispielsweise mittels ionisierender Strahlung.

Vorzugsweise können diese "Rohdispos" als Bandware vorliegen, die in Einzeldispos aufgeteilt wird, beispielsweise durch Schneiden oder Stanzen.
(5) Aufbringen (Sputtern, Drucken etc.) oder Einbetten (Ablasern, Ätzen, Spritzguss etc.) der Elektroden in das Gehäuse.
(6) Einbringen des Testelements mit Nachweiselement in das Gehäuse auf die Elektroden.
(7) Testmontage, d. h. Verbinden des "Rohdispos" mit dem Gehäuse.
(8) Verschließen und Verpacken des Gehäuses

Unabhängig davon, ob die erfindungsgemäßen "Rohdispos" als Rollen- oder Bandware in einem kontinuierlichen Prozess oder batchweise oder einzeln gefertigt werden, können Lanzette und Gehäuse vor oder nach der Sterilisation der Lanzette miteinander verbunden werden. Eine Sterilisation nach der Montage hat zur Folge, dass die Testchemie bei der Sterilisation ausreichend abgedeckt wird, da sonst eine Schädigung der Reagenzien eintreten kann.

Schließlich ist Gegenstand der Erfindung die Verwendung eines Kunststoff Materials als Bestandteil einer Lanzette eines analytischen. Hilfsmittels, wobei das kunststoff Material dazu dient, die Sterilität zumindest der Spitze einer Lanzettennadel im unbenutzten Zustand zu erhalten.

Die erfindungsgemäße Verwendung eines elastischen Materials zum Abschirmen der Spitze der Lanzettennadel erlaubt es, die Sterilität einer unbenutzten Lanzettennadelspitze zu gewährleisten.

Die Sterilität der Lanzettennadelspitze im unbenutzten Zustand ist durch geeignete Maßnahmen, wie beispielsweise Behandlung mit ionisierender Strahlung, herzustellen. Einmal sterilisiert bleiben die Lanzettennadelspitzen durch die entsprechenden Schutzkappen, die unter anderem ein elastisches Material beinhalten, erhalten.

Die erfindungsgemäße Verwendung der Schutzkappe, ermöglicht zusätzlich die Abdichtung mindestens einer Öffnung der Testkammer. Diese mindestens eine Öffnung ist gleichzeitig die Austrittsöffnung für die Lanzette und die Eintrittsöffnung für die Körperflüssigkeit.

Die Erfindung weist die folgenden Vorteile auf:
- Die Spitze der Lanzettennadel ist bei allen Ausführungsformen im unbenutzten Zustand keimdicht abgeschirmt, das heißt, Keime können bis unmittelbar vor Benutzung der Lanzette nicht an die Lanzettenspitze vordringen. Nach geeigneter Sterilisierung bleiben die Lanzettenspitzen über lange Zeit steril.
- Die Sterilität der Lanzettenspitze wird auch bei nachfolgenden Fertigungsschritten, wie zum Beispiel dem Verbinden von Lanzette und Testelement, gewährleistet. Dabei ist die empfindliche Nadelspitze vor mechanischen Einflüssen (Verbiegung etc.) geschützt.
- Der Benutzer der erfindungsgemäßen Dispos ist vor unbeabsichtigter Verletzung an einer unbenutzten Lanzettennadel geschützt. Gleiches gilt natürlich für andere Personen als den eigentlichen Benutzer.
- die Testkammer ist vor Benutzung der Lanzette abgedichtet.
- alle Öffnungen der Testkammer sind durch die Schutzkappe allein, oder in Kombination mit einer weiteren Dichtung verschlossen.
- Die erfindungsgemäßen Dispos sind kostengünstig in großen Stückzahlen mit herkömmlichen Herstellverfahren herzustellen.
- Die erfindungsgemäßen Dispos sind weitgehend miniaturisierbar und eignen sich deshalb für den Einsatz in kompakten, automatisierten Systemen.
- Als analytische Testelemente können bekannte Varianten von elektrochemischen oder optischen Sensoren eingesetzt werden.
- bei elektrochemischer Messung kann die Lanzette (wenn sie aus leitfähigem Material gefertigt ist) als Füllstandsmesser und/oder Gegenelektrode benutzt werden.

### Kurzbeschreibung der Figuren

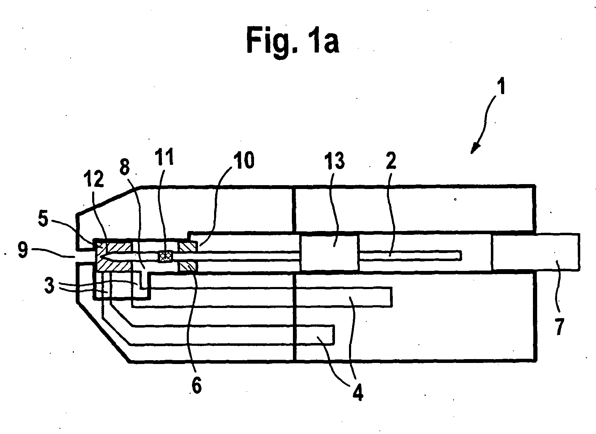

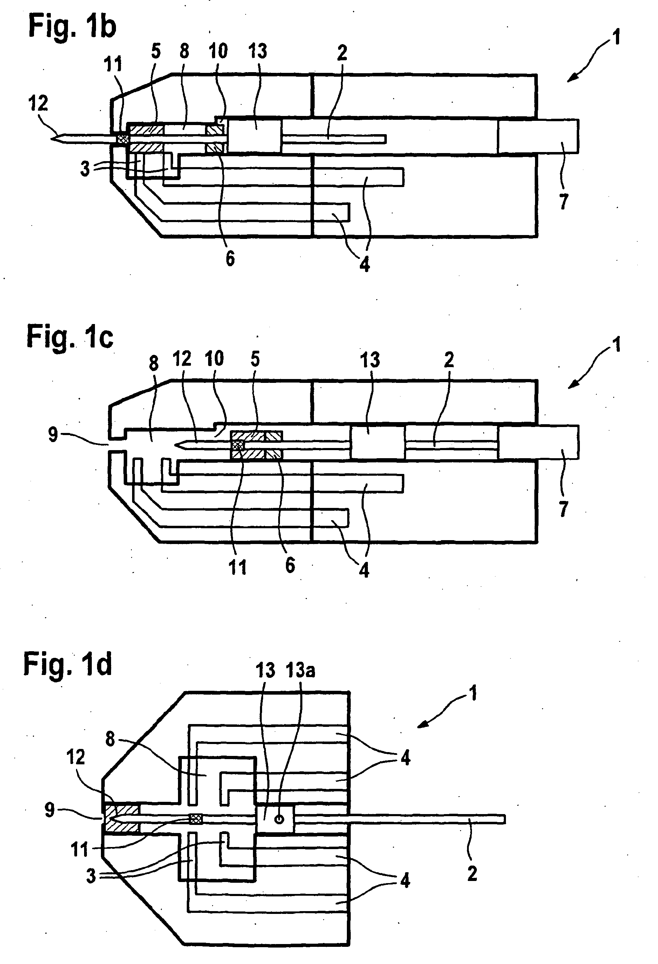

Figur 1 (a-c): Schematische Darstellung des Dispos in Seitenansicht im unbenutzten Zustand, während der Aktion und im Messzustand mit 2 Elektroden
Figur 1 d: Schematische Darstellung des Dispos in Aufsicht vor der Benutzung mit 4 Elektroden.
Figur 2 (a - d):Schematische Darstellung einer Fangeinrichtung (aufgeraute Fläche) an der Lanzettennadel vor (a) und nach (b) der Benutzung sowie vor (c) und nach (d) Benutzung mit zusätzlicher Dichtung.

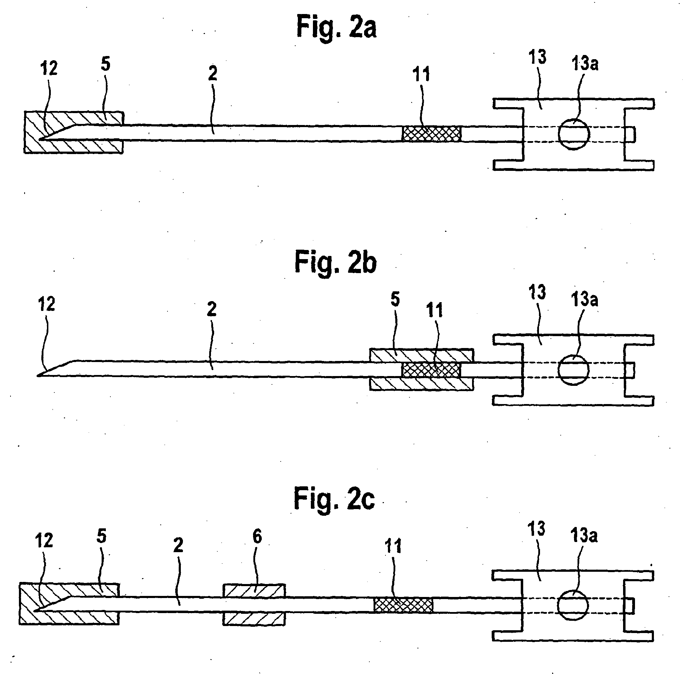

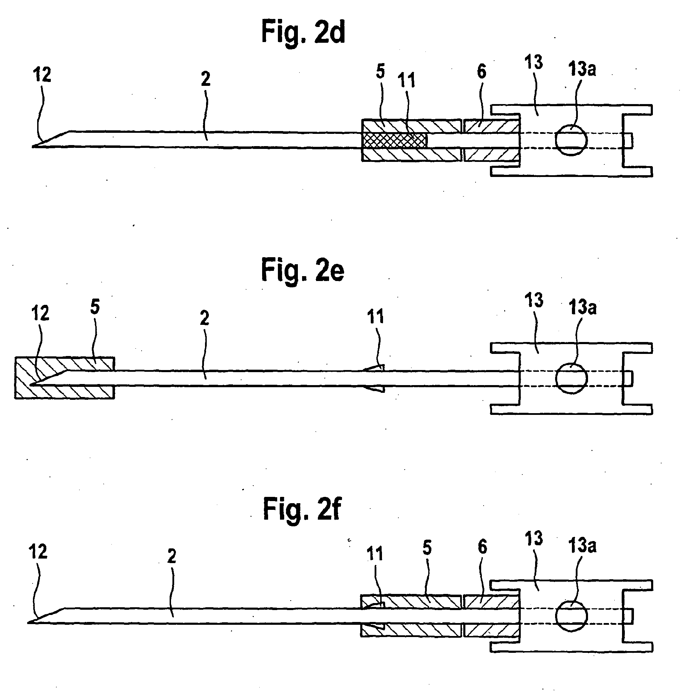

Figur 2 (e, f): Schematische Darstellung einer weiteren Fangeinrichtung (Fangborsten) an der Lanzettennadel, vor (e) und nach (f) Benutzung.

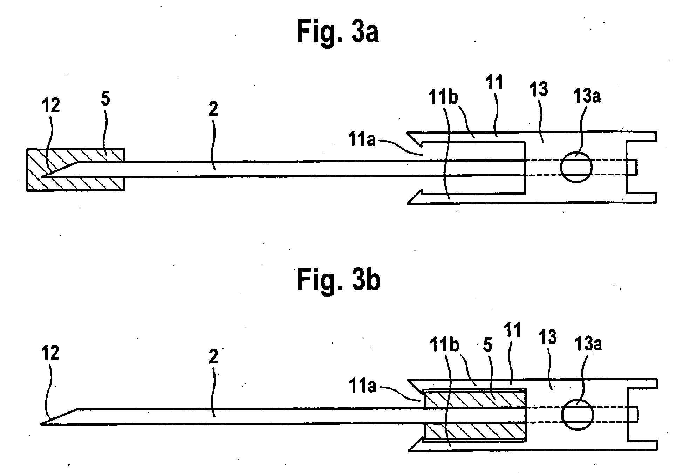

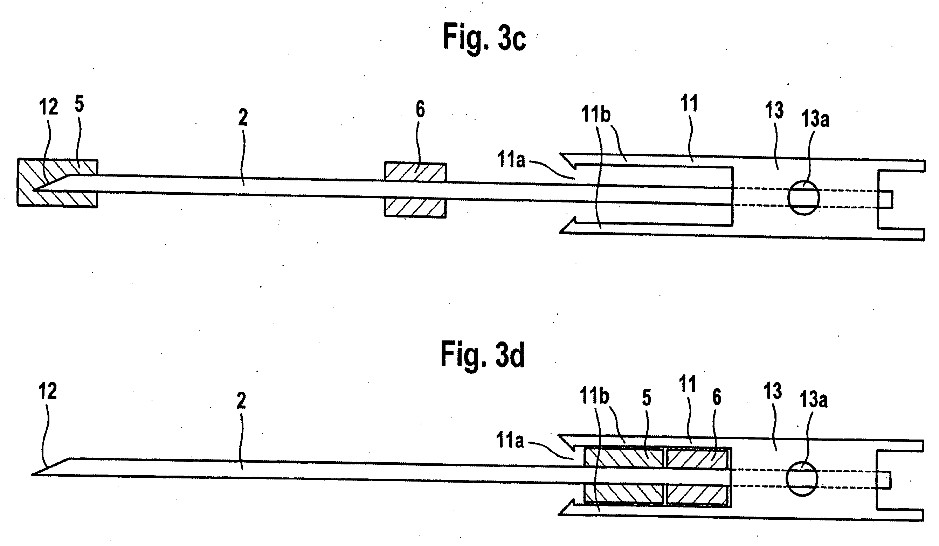

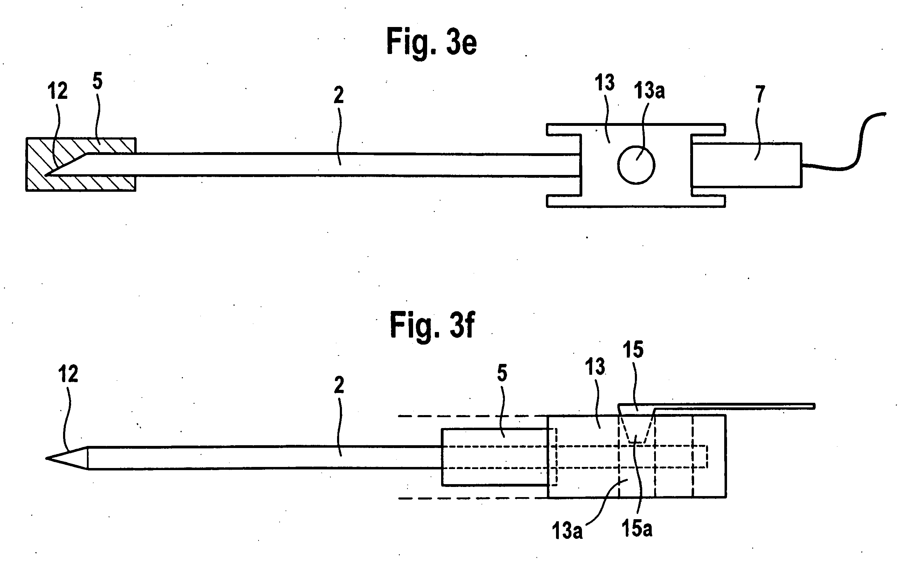

Figur 3 (a-d): Schematische Darstellung der Lanzette mit Schutzkappe und Fanghaken (a, b) sowie Schutzkappe, Dichtung und Fanghaken (c, d) sowohl im Ruhezustand (a, c) als auch im benutzten Zustand (b, d).
Figur 3 (e, f): Schematische Darstellung der Kontaktierung über die Lanzettenhaltung in Seitenansicht vor Aktuation (a) und in Aufsicht nach Aktuation (b).

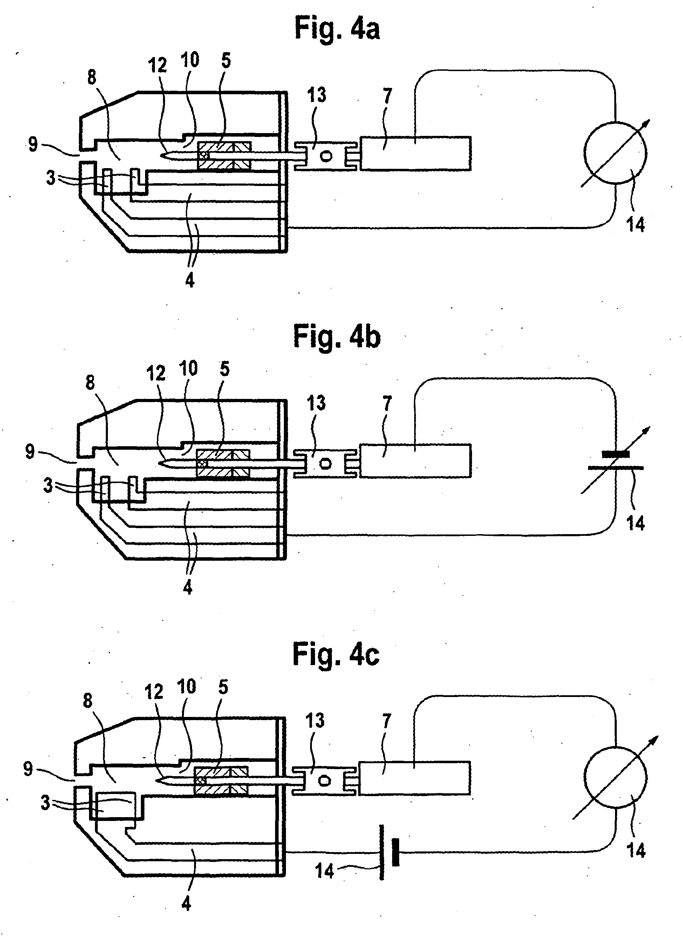

Figur 4 (a-c): Schematische Darstellung der Verwendung der Lanzette als Füllstandskontrolle (a) oder Gegenelektrode (b), oder in Kombination von Füllstandsmessung und Gegenelektrode (c).

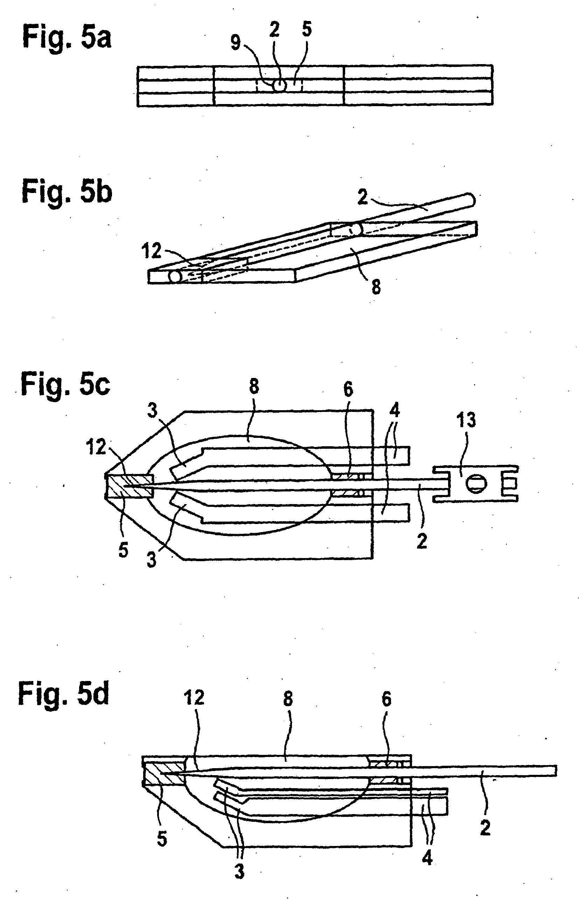

Figur 5 (a-d): Schematische Darstellung verschiedener Kammergeometrien wie quaderförmig (a), ovale Vollkammer (b) oder ovale Halbkammer (c).

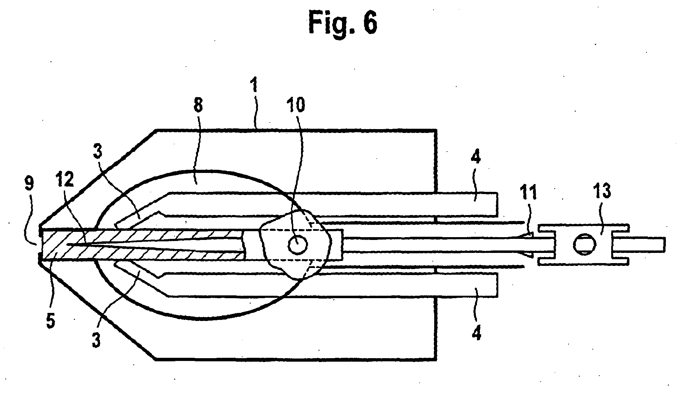

Figur 6: Schematische Darstellung der Schutzkappe mit zusätzlicher Dichtungsfunktion einer zweiten Öffnung.

### Beschreibung der Figuren

In Figur 1 a-c wird das Dispo in drei verschiedenen Aktionszuständen gezeigt. Figur 1 a zeigt das Dispo (1) mit der Lanzette (2) in unbenutztem Zustand. Die Spitze (12) der Lanzette ist dabei von einer Schutzkappe (5) umgeben. Die Lanzettenspitze (12) befindet sich dabei mit mindestens einer Elektrode (3) in einer Testkammer (8). Kontakte (4) bilden eine Verbindung der Elektroden (3) mit einem Messgerät. Die Testkammer (8) besitzt mindestens zwei Öffnungen (9, 10) wobei die Öffnung (9) durch die Schutzkappe (5) abgedichtet wird und die Öffnung (10) entweder ebenfalls durch die Schutzkappe (5) abgedichtet wird oder durch eine weitere Dichtung (6). In Figur 1 b wird das Dispo in Benutzung gezeigt. Die Halterung (13) weist an einer Seite eine Aussparung (13a) auf, die dazu dient eine Verbindung mit einem Antrieb zu gewährleisten (hier nicht gezeigt). Hierbei tritt die Lanzette (2) durch die Öffnung (9) aus dem Gehäuse aus. Figur 1 c zeigt die Lanzette (2) nach ihrer Benutzung im Dispo (1). Die Lanzette (2) hat dabei sowohl die Schutzkappe (5) als auch die Dichtung (6) soweit mit zurückgezogen, dass sowohl die Öffnung (9) als auch die Öffnung (10) freigegeben werden. Das proximale Ende der Lanzette tritt in diesem Zustand mit einem Kontakt (7) in Verbindung. In Figur 1 d ist ein Dispo (1) mit mehreren Elektroden (3) dargestellt. In diesem Fall sind es 4 Elektroden, dies können aber auch mehr sein. Dies dient dazu, Kontrollmessungen durchzuführen oder mehr als einen Analyten in der Probeflüssigkeit zu messen. In diesem Ausführungsbeispiel ist die Geometrie der Testkammer verändert, sodass Lanzettenhalterung (13) zusätzlich eine Aussparung (13a) aufweist, die nach Aktuation der Lanzette die Belüftung der Testkammer gewährleistet. In dieser Ausführungsform besitzt die Halterung (13) einen Belüftungskanal von der Aussparung (13a) zur Testkammer (hier nicht gezeigt). Vor der Aktuation wird die Sterilität der Testkammer durch eine Versiegelung dieser Aussparung (13a) gewährleistet, die bei Aktuation beseitigt oder verletzt wird (hier nicht gezeigt).

Figuren 2 a - f zeigen ebenfalls die Lanzette (2) in verschiedenen Aktuationszuständen. Hier ist das Prinzip der Fangeinrichtung (11) unter Darstellung verschiedener Fangmöglichkeiten dargestellt. In der Figur 2 a wird die Lanzette (2) mit einer Fangeinrichtung (11) im Ruhezustand gezeigt. Die Fangeinrichtung befindet sich zwischen dem proximalen und dem distalen Ende der Lanzette (2). Sie ist so angeordnet, dass sie bei Aktuation der Lanzette (2), die Schutzkappe (5) erreicht, da die Schutzkappe (5) einen größeren Durchmesser besitzt als die Gehäuseöffnung (9) und somit die Schutzkappe. (5) am weiteren vorwärts Bewegen gehindert wird. Die Fangeinrichtung (11) erreicht bei maximaler Auslenkung der Lanzette (2) die Schutzkappe (5) und beim Zurückziehen der Lanzette (2) wird die Schutzkappe (5) aufgrund des Zusammenwirkens von Fangeinrichtung (11) und Schutzkappe (5) mit zurückgezogen. Die Fangeinrichtung (11) ist vorzugsweise eine aufgeraute Fläche an der aufgrund von Reibungskräften die Schutzkappe (5) wie auch eine zweite Dichtung (6) hängen bleiben, wie in Figuren 2 a bis 2 d dargestellt. Die zweite Dichtung (6), wie sie in Figur 2c dargestellt ist, dient dazu eine zweite Öffnung (10) der Testkammer zu verschließen bevor die Lanzette aktuiert wird. Eine weitere Ausführungsform der Fangeinrichtung (11) ist in Figur 2 e abgebildet. Hier ist die Fangeinrichtung (11) durch Fangborsten realisiert. Eine weitere Ausführungsform der Fangeinrichtung (11) ist ein Fanghaken wie er schematisch in Figur 3a gezeigt wird. Der Fanghaken ist mittels einer Halterung (13) an der Lanzette fixiert und weist eine Öffnung (11a) in Richtung der Lanzettespitze (12) auf. Die Öffnung (11a) ist der Gestalt, dass sie die Schutzkappe (5) bei maximaler Auslenkung der Lanzette (2) ergreift und beim Zurückziehen der Lanzette (2) ebenfalls mit zurück befördert. Der Fanghaken besitzt mindestens einen Arm (11b) an dessen distalem Ende ein Widerhaken ist, der sich bei Aktuation der Lanzette (2) über die Schutzkappe (5) schiebt und somit die Schutzkappe (5) einfängt. Figuren 3b und 3d zeigen diese Fangeinrichtung nach Aktuation der Lanzette (2). In diesen Darstellungen ist der Fanghaken mit zwei Fangarmen (11b) ausgestattet und kann somit die Schutzkappe (5) sowie die Dichtung (6) von zwei Seiten umschließen. Der Fanghaken kann aber auch mehr als 2 Fangarme (11b) besitzen. In den

Figuren 3c und 3d ist dieses Fanghakenprinzip mit mindestens einem Arm (11b) für den Fall einer zusätzlichen Dichtung (6) gezeigt. Hierbei zeigt Figur 3c die Lanzette im Ruhezustand vor der Aktuation und in Figur 3d ist die Lanzette nach der Aktuation mit zurückgezogener Schutzkappe(5) und Dichtung (6) gezeigt.

Die Figuren 3e und 3f zeigen die Kontaktierung der Lanzette (2) nach der Aktuation. In diesem Zustand greift ein Federkontakt (7) in eine Aussparung (13a) der Lanzettenhalterung (13) ein. Dabei tritt die abgeknickte Spitze (15 a) des Federkontaktes (7) mit der Lanzette (2) in Kontakt. Die Verwendung der Lanzette (2) als Füllstandskontrolle oder Gegenelektrode ist Gegenstand der Figur 4a bis 4c. Hierbei tritt die Lanzette(2) nach Aktuation mit einem Federkontakt (7) in Verbindung.

Zur Füllstandsmessung, abgebildet in Figur 4 a, wird das sich ändernde Potenzial zwischen Lanzette (2) und Elektrode (3) gemessen. Die Befüllung der Testkammer (8) kann auch durch Messen eines Stromes bei Anlegen einer Gleich- oder Wechselspannung detektiert werden. Zusätzlich kann die Lanzettennadel als Messelektrode benutzt werden, was in Figur 4 b dargestellt ist. Auch hier tritt die Lanzette (2) nach Rückzug mit einem Kontakt (7) in Verbindung und es wird eine Gleich- oder Wechselspannung (14) zwischen Arbeitselektrode (3) in der Kammer und der Lanzette (2) angelegt. Die Änderung des Stromflusses durch die Reaktion der Testflüssigkeit mit der Testchemie auf der Elektrode (3) kann auf diese Art und Weise gemessen werden. In diesem Fall wird nur eine Elektrode in der Testkammer benötigt. Figur 4 c zeigt eine Kombination der beiden Zusatzeigenschaften Füllstandsmessung und Gegenelektrode der Lanzette (2). Hierbei werden 2 getrennte Stromkreise (14) an Lanzette (2) und Elektrode (3) angelegt.

Figur 5a zeigt eine Frontansicht des Dispos (1) wobei in der Öffnung (9) die Schutzkappe (5) mit der Lanzette (2) angeordnet ist. In Figur 5b, die die Testkammer in seitlicher Ansicht zeigt, ist der Verlauf der Lanzette (2) in der Testkammer (8) zu erkennen.

Fig. 5c zeigt eine ovale Testkammer, die sich über den Elektroden bis hin zur Schutzkappe (5) erstreckt. Eine alternative Kammer ist in Figur 5d abgebildet, wobei die ovale Kammer durch eine Abgrenzung an der den Elektroden gegenüberliegenden Seite begrenzt ist und dadurch ein kleineres Kammervolumen verwirklicht wird.

Figur 6 zeigt ein System bei dem die Schutzkappe (5) zusätzlich als Verschlussmechanismus für die zweite Öffnung (10) dient, die in diesem Fall ein Entlüftungsloch in der Deckelfolie ist. Zu erkennen sind in diesem System die Elektroden (3), die in die Kammer (8) hineinreichen, sowie die Schutzkappe (5), die die Kammer und die Lanzettenspitze (12) abdichtet sowie einen weiteren Teil der Lanzette (3) umschließt. Am proximalen Ende der Lanzette (3) befindet sich die Lanzettenhalterung (13). Vor der Lanzettenhalterung ist die Fangeinrichtung (11) in Richtung distalem Ende der Lanzette (3) angeordnet.

## Patentansprüche

1. Analytisches Hilfsmittel, enthaltend
eine Lanzette (2), umfassend
eine Lanzettennadel mit einer Spitze (12) und
einer Schutzkappe (5), die die Lanzettennadel zumindest im Bereich der Spitze (12) vollständig umgibt,
wobei die Lanzettennadel während des Stechvorgangs relativ zur Schutzkappe (5) verschiebbar ist und die Lanzette (2) so mit der Schutzkappe (5) zusammenwirkt, dass die Lanzette (2) bei ihrem Zurückziehen die Schutzkappe (5) mit zurückzieht, **dadurch gekennzeichnet, dass** das analytische Hilfsmittel ein Testelement mit einer Kammer (8), umfassend ein Reagenzsystem, enthält, wobei die Kammer (8) eine Öffnung (10) aufweist, die von der Schutzkappe (5) im Ruhezustand vor der Benutzung verschlossen wird.

2. Analytisches Hilfsmittel gemäß Anspruch 1, **dadurch gekennzeichnet, dass** das Material der Schutzkappe (5) ein Elastomer ist.

3. Analytisches Hilfsmittel gemäß Anspruch 1 oder 2, **dadurch gekennzeichnet, dass** die Schutzkappe (5) die Nadelspitze steril umschließt.

4. Analytisches Hilfsmittel gemäß einem der Ansprüche 1 bis 3, **dadurch gekennzeichnet, dass** das analytische Hilfsmittel Mittel zum Probenflüssigkeitstransport aufweist.

5. Analytisches Hilfsmittel gemäß Anspruch 4, **dadurch gekennzeichnet, dass** das Mittel zum Probenflüssigkeitstransport ein Kapillarspalt, ein Kapillarkanal oder ein Docht aus einem saugfähigen Material oder ein Steg aus einem saugfähigen Material ist.

6. Analytisches Hilfsmittel gemäß einem der Ansprüche 1 bis 5, **dadurch gekennzeichnet, dass** das Hilfsmittel eine zweite Öffnung (9) besitzt, die durch eine, relativ zur Lanzette (2) und zum Hilfsmittel verschiebbare Dichtung (6) abgedichtet wird.

7. Analytisches Hilfsmittel gemäß Anspruch 6 **dadurch gekennzeichnet, dass** die Schutzkappe (5) beide Öffnungen (9, 10) abdichtet.

8. Analytisches Hilfsmittel gemäß einem der Ansprüche 1 bis 7 **dadurch gekennzeichnet, dass** sich an der Lanzette (2) eine Fangeinrichtung (11a, b) oder eine Oberflächenstruktur (11) befindet, die beim Zurückziehen der Lanzette (2), die von der Lanzette (2) durchstoßene Schutzkappe (5) mit zurückzieht.

9. Analytisches Hilfsmittel gemäß Anspruch 8 **dadurch gekennzeichnet, dass** beim Zurückziehen der Lanzette (2), die zurückgezogene Schutzkappe (5), die zweite, relativ zur Lanzette (2) verschiebbare Dichtung (6), mitzieht und eine Entlüftung geöffnet wird.

10. Analytischen Hilfsmittel gemäß einem der Ansprüche 1 bis 9, **dadurch gekennzeichnet, dass** die Lanzette (2) Mittel zur Probenflüssigkeitsstandsmessung aufweist und/oder als Gegenelektrode dient.

11. Verfahren zur Herstellung eines analytischen Hilfsmittels, mit den Schritten
- Bereitstellen einer Lanzette (2), bei der sich die Spitze (12) einer Lanzettennadel in einer Schutzkappe (5) befindet und
- Bereitstellen einer Fangeinrichtung an der Lanzette (2), die die Schutzkappe (5) beim Zurückziehen der Lanzette (2) mit zurückzieht,
- Bereitstellen eines Testelements mit einer Kammer (8) , enthaltend ein Reagenzsystem und eine Öffnung (10) in die Kammer (8), und
- Sterilisieren der Lanzette (2),
- Einbringen der Lanzette (2) in die Kammer (8), sodass die Schutzkappe (5) die Öffnung (10) der Kammer (8) verschließt.

12. Ein Analysesystem, mit einem analytischen Hilfsmittel beinhaltend
eine Lanzette (2), umfassend
eine Lanzettennadel mit einer Spitze (12) und
mindestens einer Schutzkappe (5), die die Lanzettennadel zumindest im Bereich der Spitze (12) vollständig umgibt, **gekennzeichnet durch**
eine Fangeinrichtung (11a, b) oder eine aufgeraute Oberfläche (11) an der. Lanzette (2), die bei Kontakt mit der Schutzkappe eine ausreichende Reibungswirkung aufweist und die die Schutzkappe (5) beim Zurückziehen der Lanzette (2) mit zurückzieht, sowie eine Detektionseinheit, die die bei der Reagenzienumsetzung entstehenden Signale detektiert, und
ein Auswertegerät, das aus den Signalen eine Konzentration des Analyten bestimmt.

## Claims

1. Analytical device containing
a lancet (2) comprising
a lancet needle with a tip (12) and
a protective cap (5) which completely surrounds the lancet needle at least in the area of the tip (12),
wherein the lancet needle can be displaced relative to the protective cap (5) and the lancet (2) interacts with the protective cap (5) in such a manner that the lancet (2) pulls the protective cap (5) back along with it when it is retracted, **characterized in that** the analytical device contains a test element with a chamber (8) containing a reagent system, the chamber (8) having an opening (10) that is sealed by the protective cap (5) in the resting state before use.

2. Analytical device according to claim 1, **characterized in that** the material of the protective cap (5) is an elastomer.

3. Analytical device according to claim 1 or 2, **characterized in that** the protective cap (5) encloses the needle tip in a sterile manner.

4. Analytical device according to one of the claims 1 to 3, **characterized in that** the analytical device has means for transporting sample liquid.

5. Analytical device according to claim 4, **characterized in that** the means for transporting sample liquid is a capillary gap, a capillary channel or a wick made of an absorbent material or a cross-piece made of an absorbent material.

6. Analytical device according to one of the claims 1 to 5, **characterized in that** the device has a second opening (9) which is sealed by a seal (6) that can be displaced relative to the lancet (2) and to the device.

7. Analytical device according to claim 6, **characterized in that** the protective cap (5) seals both openings (9, 10).

8. Analytical device according to one of the claims 1 to 7, **characterized in that** a catching device (11a, b) or a surface structure (11) is located on the lancet which pulls the protective cap (5) pierced by the lancet (2) back along with it when the lancet (2) is retracted.

9. Analytical device according to claim 8, **characterized in that** when the lancet (2) is retracted, the retracted protective cap (5) pulls the second seal (6) that can be displaced relative to the lancet along with it and an air vent is opened.

10. Analytical device according to one of the claims 1 to 9, **characterized in that** the lancet (2) has means for measuring the level of sample liquid and/or serves as a counter electrode.

11. Process for producing an analytical device comprising the steps
- providing a lancet (2) in which the tip (12) of a lancet needle is located in a protective cap (5) and
- providing a catching device on the lancet (2) which pulls the protective cap (5) back along with it when the lancet (2) is retracted,
- providing a test element with a chamber (8) containing a reagent system and an opening (10) in the chamber (8) and
- sterilizing the lancet (2),
- introducing the lancet (2) into the chamber (8) such that the protective cap (5) seals the opening (10) of the chamber (8).

12. An analytical system with an analytical device comprising a lancet (2) comprising
a lancet needle with a tip (12) and
at least one protective cap (5) which completely surrounds the lancet needle at least in the area of the tip (12), **characterized by** a catching device (11a, b) or a roughened surface (11) on the lancet (2) which on contact with the protective cap has an adequate frictional effect and which pulls back the protective cap (5) along with it when the lancet (2) is retracted, as well as a detection unit which detects signals that are generated during the reagent reaction and
an evaluation device which determines a concentration of the analyte from the signals.

## Revendications

1. Instrument d'analyse, comportant
une lancette (2), comprenant
une aiguille-lancette équipée d'une pointe (12) et
d'un capuchon protecteur (5) entourant complètement l'aiguille-lancette au moins au niveau de la pointe (12),
l'aiguille-lancette étant mobile par rapport au capuchon protecteur (5) pendant le processus de piqûre et la lancette (2) coopérant avec le capuchon protecteur (5) de telle sorte que la lancette (2) retire le capuchon protecteur (5) avec elle lors de son retrait, **caractérisé en ce que** l'instrument d'analyse comporte un élément d'essai équipé d'une chambre (8) comprenant un système réactif, la chambre (8) présentant une ouverture (10) qui est fermée par le capuchon protecteur (5) à l'état de repos avant l'utilisation.

2. Instrument d'analyse selon la revendication 1, **caractérisé en ce que** le matériau du capuchon protecteur (5) est un élastomère.

3. Instrument d'analyse selon la revendication 1 ou 2, **caractérisé en ce que** le capuchon protecteur (5) entoure de manière stérile la pointe d'aiguille.

4. Instrument d'analyse selon une des revendications 1 à 3, **caractérisé en ce que** l'instrument d'analyse présente des moyens pour le transport de liquide d'essai.

5. Instrument d'analyse selon la revendication 4, **caractérisé en ce que** le moyen de transport de liquide d'essai est une fente capillaire, un canal capillaire ou une mèche composée d'un matériau absorbant ou une traverse composée d'un matériau absorbant.

6. Instrument d'analyse selon une des revendications 1 à 5, **caractérisé en ce que** l'instrument comporte une seconde ouverture (9) rendue étanche par un joint (6) mobile par rapport à la lancette (2) et à l'instrument.

7. Instrument d'analyse selon la revendication 6, **caractérisé en ce que** le capuchon protecteur (5) rend étanche les deux ouvertures (9, 10).

8. Instrument d'analyse selon une des revendications 1 à 7, **caractérisé en ce qu'**un dispositif de capture (11a, b) ou une structure de surface (11) se trouve au niveau de la lancette (2) et, lors du retrait de la lancette (2), retire avec elle le capuchon protecteur (5) transpercé par la lancette (2).

9. Instrument d'analyse selon la revendication 8, **caractérisé en ce que**, lors du retrait de la lancette (2), le capuchon protecteur (5) retiré retire avec lui le second joint (6) mobile par rapport à la lancette (2), ce qui ouvre une aération.

10. Instrument d'analyse selon une des revendications 1 à 9, **caractérisé en ce que** la lancette (2) présente des moyens de mesure du niveau de liquide d'essai et/ou sert de contre-électrode.

11. Procédé de fabrication d'un instrument d'analyse, comprenant les étapes de
- voir une lancette (2), dans laquelle la pointe (12) d'une aiguille-lancette se trouve dans un capuchon protecteur (5) et
- voir un dispositif de capture au niveau de la lancette (2) qui, lors du retrait de la lancette (2), retire avec elle le capuchon protecteur (5),
- voir un élément d'essai équipé d'une chambre (8), comportant un système réactif et une ouverture (10) dans la chambre (8), et
- stériliser la lancette (2),
- insérer la lancette (2) dans la chambre (8) de sorte que le capuchon protecteur (5) obture l'ouverture (10) de la chambre (8).

12. Système d'analyse, équipé d'un instrument d'analyse comportant
une lancette (2), comprenant
une aiguille-lancette équipée d'une pointe (12) et
au moins d'un capuchon protecteur (5) entourant complètement l'aiguille-lancette au moins au niveau de la pointe (12), **caractérisé par** un dispositif de capture (11a, b) ou une surface rugueuse (11) au niveau de la lancette (2), qui présente une action de frottement suffisante lors du contact avec le capuchon protecteur et qui,
lors du retrait de la lancette (2), retire avec elle le capuchon protecteur (5), ainsi que
une unité de détection, qui détecte les signaux produits lors de la transformation des réactifs, et
un appareil d'évaluation, qui détermine une concentration d'analyte à partir des signaux.
